# FASCICULE DE BREVET EUROPEEN

(11) **EP 0 676 052 B1**
(45) Date de publication et mention de la délivrance du brevet: **12.04.2000**
(21) Numéro de dépôt: 94902859.1
(22) Date de dépôt: 23.12.1993
(51) Int. Cl.: G01N 33/68, C07K 14/00

(54) **DETECTION DE LA MUCOVISCIDOSE OU D'UNE MUTATION DU GENE CFTR**
FESTSTELLUNG DER CYSTIC FIBROSIS ODER EINER MUTATION DES CFTR GENS
DETECTION OF CYSTIC FIBROSIS OR A CFTR GENE MUTATION

(30) Priorité: 24.12.1992 FR 9215730
(43) Date de publication de la demande: 11.10.1995
(73) Titulaire: INSTITUT NATIONAL DE LA SANTE ET DE LA RECHERCHE MEDICALE (INSERM), 75654 Paris Cédex 13 (FR)
(72) Inventeur: IOVANNA, Juan-Lucio, F-13009 Marseille (FR); DAGORN, Jean-Charles, F-13009 Marseille (FR); KEIM, Volker, D-6805 Heddesheim (DE); SARLES, Jacques, F-13420 Gemenos (FR)
(74) Mandataire: Desaix, Anne
(86) Numéro de dépôt international: FR9301299
(87) Numéro de publication internationale: WO9415218

(56) Documents cités:
- EP-A- 0 446 017
- WO-A-91/02796
- WO-A-91/16428
- US-A- 4 322 274
- GASTROENTEROLOGY vol. 103, no. 1 , Juillet 1992 pages 248 - 254 V.KEIM ET AL. 'A Novel Exocrine Protein Associated With Pancreas Transplantation in Humans.' cité dans la demande
- PEDIATRIC PULMONOLOGY vol. 7 , 1991 pages 11 - 18 P.M.FARRELL ET AL. 'Current Issues in Neonatal Screening for Cystic Fibrosis and Implications of the CF Gene Discovery.' cité dans la demande
- International Society for Neonatal Screening, "PAP assay for CF Neonatal Screening, a prospective evaluation on 210.000 newborns", Sarles J., oral presentation, Boston, oct. 21-24, 1996

## Description

La présente demande a pour objet le dépistage de la mucoviscidose ou d'une mutation du gène responsable de la mucoviscidose associée à une affection pancréatique, au moyen d'un dosage de la PAP (Pancreatitis-Associated Protein)

La PAP a été isolée, purifiée et caractérisée chez l'homme et décrite dans la demande de brevet PCT publiée le 31 Octobre 1991 sous le n° 91/16428. Dans cette demande antérieure, la PAP a été proposée comme moyen de détection d'une pathologie déterminée, la pancréatite aiguë.

Les inventeurs ont à ce jour mis en évidence que des anomalies génétiques susceptibles de donner lieu à des affections caractéristiques de la mucoviscidose, peuvent être corrélées de façon fiable chez l'homme, avec une expression anormale de la PAP et ce dès la naissance.

La mucoviscidose, encore appelée "cystic fibrosis" en anglais est une maladie génétique très fréquente chez certaines populations, qui se caractérise par une insuffisance globale des sécrétions exocrines du pancréas et du poumon et d'une manière générale, des glandes exocrines. Cliniquement, la maladie est associée à des sécrétions trop visqueuses, le mucus formé pouvant obstruer les bronches et provoquer des troubles graves ou mortels.

Le gène de la mucoviscidose a été localisé sur le chromosome 7 humain. Ce gène, appelé gène CFTR ("cystic fibrosis transmembrane conductance regulator") présente des mutations dans différentes régions, chez les sujets atteints de mucoviscidose. Des mutations du même type peuvent être détectées sur un seul des deux chromosomes 7, chez des sujets dits "porteurs" mais ne présentant pas de signes cliniques de la maladie. Ces personnes sont hétérozygotes pour la mutation du gène CFTR.

Dans le cas d'une mutation hétérozygote, le sujet porteur peut toutefois souffrir de certains troubles caractéristiques d'une altération des glandes sécrétoires. Le sujet porteur peut par exemple être atteint de troubles au niveau du pancréas.

Le diagnostic de la mucoviscidose a en premier lieu été etfectué par un test appelé "test à la sueur" consistant à doser le chlore et le sodium dans la sueur de sujets en particulier d'enfants susceptibles d'être atteints par cette maladie. L'indication d'un taux de chlore situé entre 60 et 180 mEq/l pouvait être corrélée avec la maladie, dans la mesure où ce taux est d'environ 40 mEq/l chez le nourrisson normal.

Plusieurs tests d'un autre type ont été successivement proposés (Berry, H.K. et al, Am.J.Dis.Child. 1980 134:930 ; Crossley, J.R., et al, Lancet 1977 ii:1093 ; Forrest, D.C., et al Arch.Dis.Child. 1981 56:156 ; Green, M.N. et al, Pediatrics 1968 41:989 ; Robinson, P.G. et al Arch.Dis.Child. 1976 51:301 ; Shwachman, H., et al, Pediatrics 1949 4:222), mais seul le dosage sérique de trypsine chez le nouveau-né (Farriaux, J.P., et al, Immunoanal.Biol.Spéc. 1992 33:71) a démontré suifisamment d'intérêt pour être encore en vigueur dans certains pays (Farrell, P.M., et al, Ped.Pulmonol. 1991 Supplement 7:11). Ce dosage radioimmunologique est réalisé sur des taches de sang déposé sur carton, prélevé chez les nouveau-nés aux fins de dépistage des autres maladies génétiques actuellement dépistées systématiquement, la phénylcétonurie et l'hypothyroïdisme. Le dépistage par la trypsine sérique reste cependant très imparfait puisque les résultats d'un programme français d'évaluation à grande échelle ont récemment amené l'Association Française de Dépistage à ne pas le rendre obligatoire (Farriaux, J.P., et al, Immunoanal.Biol.Spéc. 1992 33:71).

Le principal problème posé par le dosage sérique de trypsine est celui des faux-positifs (environ 1 % de la population alors que l'incidence de la maladie est an France d'environ 0.03 %, (Farriaux, J.P., et al, Immunoanal.Biol.Spéc. 1992 33:71).

L'invention propose de nouveaux moyens pour réaliser un test de dépistage de la mucoviscidose ou d'une affection de certaines glandes exocrines, an particulier du pancréas, affection liée à l'existence d'une mutation hétérozygote du gène CFTR.

Les moyens de l'invention permettent de remédier de façon significative aux inconvénients présentés par les tests connus jusqu'à présent, et an particulier ces moyens offrent la possibilité de diminuer considérablement voire d'abolir statistiquement le nombre de résultats faux positifs.

La possibilité de dépister la mucoviscidose ou une affection pancréatique résultant d'une mutation du gène CFTR, grâce à la recherche de la PAP, a permis la mise au point d'un test suceptible d'être appliqué lors d'un dépistage néonatal ou lors d'un dépistage chez l'enfant ou chez l'adulte de la mucoviscidose.

Ce test peut également être réalisé dans le but de suivre l'évolution de la maladie par exemple pour déterminer l'évolution de l'affection pancréatique.

Un tel test peut également être mis an oeuvre pour détecter la présence d'un gène CFTR muté hétérozygote ne conduisant pas à l'apparition d'une mucoviscidose mais susceptible d'être corrélée à une affection du pancréas, chez un patient adulte ou enfant.

L'invention décrit donc un procédé pour la détection in vitro d'une affectation pancréatique associée à une altération du gène CFTR, caractérisé en ce que l'on dose la concentration de PAP humaine dans un échantillon biologique.

L'invention concerne plus particulièrement un procédé pour le dépistage in vitro de la mucoviscidose, caractérisé en ce qu'il comprend une étape où l'on dose la concentration de PAP humaine dans un échantillon biologique.

La valeur de la concentration obtenue peut ensuite être comparée à une valeur qui serait obtenue dans les mêmes conditions de test, on l'absence de toute pathologie ou en l'absence de mutation hétérozygote du gène CFTR.

Les inventeurs ont constaté que dans une pathologie telle que la mucoviscidose, qui n'est pas nécessairement associée, notamment chez le nourrisson, à une pancréatite aiguë même dans le cas d'une insuffisance pancréatique, on remarque une augmentation significative, voire considérable du taux de PAP par rapport au taux normal. Cette augmentation peut être de 2 à 3 fois par rapport à la valeur normale, jusqu'à 100 fois cette valeur. La valeur normale est déterminée par référence à la médiane telle que définie ci-après.

Le dosage proposé de la concentration en PAP dans un échantillon biologique, comme indication d'une altération du gène CFTR associée à une affection pancréatique ou d'une mucoviscidose, présente l'avantage d'être utilisable dans le cadre du diagnostic néonatal.

Malgré le phénomène connu et non pathologique de passage transitoire dans le sang de certaines enzymes au moment de la naissance, le dosage de la PAP reste en effet statistiquement fiable pour détecter la mucoviscidose ou une mutation hétérozygote sur le gène CFTR associée à une affection pancréatique. En d'autres termes, lorsque le taux de PAP mesuré dans un échantillon biologique, et en particulier dans le sang, lors d'un test néonatal est anormal, on peut en déduire une anomalie du gène CFTR, associée à la mucoviscidose ou dans certains cas à une altération pancréatique. Les inventeurs ont donc constaté que la présence d'un taux anormalement élevé de PAP dans le sang, lors d'un test néonatal ne peut être confondue avec la sécrétion des enzymes dans le sang qui peut accompagner le phénomène de "stress périnatal".

Par l'expression "taux anormalement élevé" de PAP on entend une valeur de la PAP par exemple supérieure à deux fois la valeur de la médiane calculée à partir du taux de PAP déterminé sur un groupe d'échantillons de référence.

Selon un premier mode de réalisation de l'invention, le procédé de dépistage in vitro de la mucoviscidose est caractérisé par :
- le dosage de la concentration de PAP dans l'échantillon biologique,
- la comparaison de la valeur obtenue, avec la valeur de la médiane, calculée pour un groupe déterminé d'échantillons de référence préalablement soumis au dosage de la PAP, dans les mêmes conditions.

Par "groupe d'échantillons de référence", on entend de préférence des échantillons obtenus chez des patients non homozygotes pour la mutation du gène CFTR.

La médiane dont il est question précédemment est la valeur de la mesure obtenue pour un échantillon donné et choisie de telle façon qu'il existe un nombre égal d'observations (mesures) inférieures et supérieures à cette valeur dans le groupe déterminé d'échantillons testés. Lorsque le nombre de mesures effectuées est pair, la médiane est indéterminée entre les deux valeurs centrales observées.

Un procédé avantageux de réalisation de l'invention est encore caractérisé en ce que le dosage de la concentration sérique de PAP comprend :
- la mise en contact d'un échantillon biologique, par exemple le sang ou le sérum, avec des anticorps reconnaissant la PAP humaine,
- la détection de la formation d'une réaction immunologique de type PAP-anticorps,
- le dosage des complexes PAP-anticorps.

Les anticorps utilisés pour la réalisation de ce dosage peuvent être des anticorps polyclonaux ou monoclonaux, voire ces deux types d'anticorps lorsque le test est un test immunologique de type sandwich.

De préférence, l'anticorps formant le complexe immunologique avec la PAP est un anticorps monoclonal. Avantageusement, il s'agit d'un anticorps monoclonal spécifique de la PAP humaine qui est par conséquent dépourvu de réaction immunologique avec les constituants présents dans l'échantillon biologique normal et en particulier dans le sang normal de référence ; cet anticorps est notamment dépourvu de réaction avec les lectines du sang.

On appelle "sang normal" ou "échantillon normal", un échantillon contenant un taux très faible de PAP.

On aura intérêt dans le cadre de la réalisation du test de dépistage in vitro de l'invention à sélectionner un anticorps ou des anticorps spécifiques de la PAP donnant lieu à un signal de bruit de fond faible (mesurable en mettant en contact un sérum normal ou un échantillon normal avec cet anticorps).

Un procédé particulièrement avantageux pour la réalisation de l'invention est caractérisé en ce qu'il comprend les étapes suivantes :
- la mise en contact d'une quantité déterminée d'un échantillon biologique, par exemple d'un échantillon de sang ou de sérum dans lequel on recherche la PAP, avec un anticorps monoclonal (dit "anticorps de capture") reconnaissant spécifiquement la PAP humaine, dans des conditions permettant la formation d'un complexe immunologique entre l'anticorps de capture et la PAP lorsqu'elle est présente dans l'échantillon dosé,
- la mise en contact du milieu de réaction obtenu à l'étape précédente, avec un anticorps monoclonal (dit "anticorps de révélation") reconnaissant un épitope différent de l'épitope reconnu par l'anticorps de capture, l'anticorps de révélation étant marqué, dans des conditions permettant la formation d'un complexe immunologique entre l'anticorps de révélation et l'antigène préalablement lié à l'anticorps de capture,
- le lavage pour éliminer les anticorps de révélation n'ayant pas réagi,
- la détection des complexes anticorps de capture - antigène PAP - anticorps de révélation,
- la détermination de la concentration de PAP, et le cas échéant sa comparaison avec une médiane calculée pour un groupe déterminé d'échantillons de référence préalablement soumis au même dosage de la PAP dans les mêmes conditions.

Pour la réalisation du procédé ci-dessus décrit, on pourra mettre en oeuvre un couple anticorps de capture/anticorps de révélation dans lequel l'anticorps de capture sera un sérum polyclonal et l'anticorps de révélation sera un anticorps monoclonal. On peut également utiliser un couple anticorps de capture/anticorps de révélation dans lequel tous les anticorps sont monoclonaux, étant entendu que l'anticorps de capture et l'anticorps de révélation reconnaissent des épitopes distincts sur la PAP.

Des résultats tout à fait satisfaisants peuvent aussi être obtenus lorsque l'anticorps de capture est un sérum polyclonal et l'anticorps de révélation est également un sérum polyclonal, de même nature et le cas échéant de même origine s'agissant de sa préparation, ce dernier étant toutefois marque.

Afin de réaliser le test ELISA de type sandwich ci-dessus décrit pour la PAP, une sélection des anticorps monoclonaux et/ou de sérums polyclonaux doit être faite, sachant que l'anticorps de capture est destiné à revêtir des puits de plaque de microtitration.

L'homme du métier tiendra compte dans la sélection de ces anticorps, du fait que l'absorption d'un anticorps sur un support solide peut conduire à une perte d'activité à des modifications conformationnelles de la molécule. Ainsi il pourra être nécessaire de vérifier dans un premier temps que l'anticorps choisi ou le sérum choisi est capable de reconnaître la PAP naturelle et/ou la PAP recombinante après avoir été fixé sur un support.

L'homme du métier sera également en mesure de sélectionner un anticorps monoclonal de révélation reconnaissant un épitope différent sur la PAP par rapport à l'épitope reconnu par l'anticorps de capture en réalisant par exemple un test de détection de la PAP par compétition entre les deux anticorps choisis.

De façon générale, le couple anticorps de capture/anticorps de révélation ou le sérum polyclonal utilisé, tant pour la capture que la révélation, doit présenter une sensibilité satisfaisante ainsi que permettre une reproductibilité des résultats intéressante et une bonne stabilité dans le temps (d'environ 6 mois pour un stockage des anticorps à 4°C).

Ainsi on mettra avantageusement en oeuvre un anticorps monoclonal de capture désigné 6F3E4, produit par l'hybridome déposé à l'ECACC (European Collection of Animal Cell Culture, Porton Down, Salisbury, Wiltshirte SP4 OJG, Grande Bretagne) sous le n° 92122310 le 23 Décembre 1992. De même, un anticorps de révélation particulièrement intéressant est l'anticorps 16F4B8 produit par l'hybridome déposé à l'ECACC sous le n°92122309 le 23 Décembre 1992.

L'anticorps ou le sérum polyclonal de révélation sera marqué avec tout marqueur approprié afin de permettre la détection du complexe anticorps de capture - antigène PAP - anticorps de révélation formé lors du test de détection. A titre indicatif, on pourra utiliser des marqueurs radioactifs ou encore des marqueurs enzymatiques. A titre d'exemple, on citera le marquage à l'aide de la peroxidase de Raifort.

Le procédé de détection dont question ci-dessus faisant appel à des anticorps, peut être modifié de telle façon que l'anticorps de capture et/ou l'anticorps de révélation sont remplacés par leurs fragments variables ou une partie de ces fragments variables. A titre d'exemple, on peut utiliser pour réaliser la réaction des fragments F(ab')₂ ou Fab.

L'invention décrit par ailleurs un anticorps monoclonal, caractérisé en ce qu'il reconnait la PAP humaine naturelle purifiée et/ou recombinante, lorsqu'il est adsorbé sur un support solide.

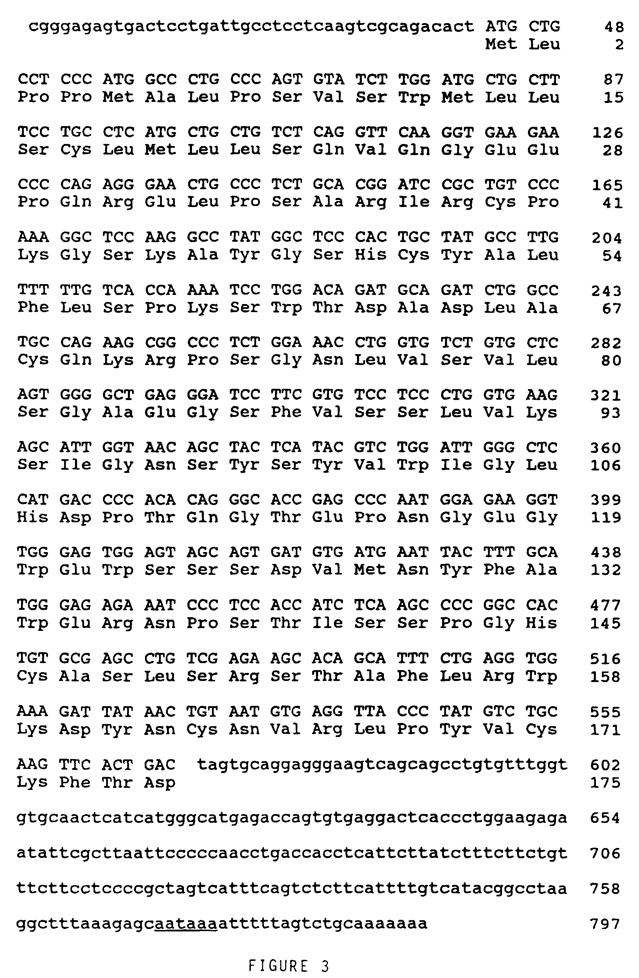

La PAP a été décrite dans la demande PCT précitée ; sa séquence nucléotidique et sa séquence en acides aminés sont rappelées à la figure 3.

Des anticorps avantageux répondant à ces conditions sont les anticorps 6F3E4 ou 16F4B8 décrits ci-dessus.

L'invention décrit également un kit comprenant au moins deux anticorps monoclonaux dirigés spécifiquement contre des épitopes différents de la PAP humaine, l'un au moins des deux anticorps (anticorps de capture) monoclonaux reconnaissant la PAP humaine lorsqu'il est fixé sur un support solide.

De manière générale, l'invention vise l'utilisation d'anticorps monoclonaux reconnaissant spécifiquement la PAP humaine, pour le dépistage in vitro de la mucoviscidose, ou d'une affection pancréatique liée à une mutation hétérozygote pour le gène CFTR.

L'invention décrit encore un kit pour le dépistage in vitro de la mucoviscidose, ou d'une affection pancréatique associée à une mutation hétérozygote pour le gène CFTR comprenant :
- des anticorps monoclonaux décrits et/ou polyclonaux ci-dessus, l'un de ces anticorps (anticorps de révélation) étant marqué,
- un réactif permettant de révéler l'anticorps de révélation,
- un témoin négatif.

Un kit préféré pour la réalisation de l'invention comprend à titre d'anticorps de capture, l'anticorps 6F3E4 et à titre d'anticorps de révélation, l'anticorps 16F4B8.

Un autre kit particulièrement intéressant contient pour la détection de la PAP, un sérum polyclonal. Une partie de ce sérum est destinée à la capture de la PAP, l'autre partie contient des anticorps marqués pour la révélation dans un test de type sandwich, de la présence d'un complexe de type PAP-anticorps.

Les sérums polyclonaux appropriés peuvent être préparés chez des animaux, par exemple des lapins, auxquels on a administré la PAP purifiée.

Un kit intéressant comprend ainsi :
- un sérum polyclonal reconnaissant la PAP humaine,
- le cas échéant un sérum polyclonal reconnaissant la PAP humaine, dont les anticorps sont marqués,
- un réactif permettant de révéler les anticorps marqués,
- un témoin négatif.

L'invention décrit aussi l'hybridome producteur de l'anticorps 6F3E4, ayant le n° 92122310 à l'ECACC, ainsi que l'hybridome producteur de l'anticorps 16F4B8, ayant le n°92122309 à l'ECACC.

D'autres caractéristiques et avantages de l'invention apparaissent dans les exemples et dans les figures qui suivent.

### Figure 1.

Résultat d'un dosage de PAP sérique au moyen d'un test de type ELISA compétitif faisant appel à des anticorps polyclonaux obtenus chez le lapin et reconnaissant la PAP humaine purifiée. Ce dosage a été réalisé chez des patients atteints de mucoviscidose.

### Figure 2.

Dosage de PAP chez le nouveau-né.

### Figure 3.

Séquence de nucléotides et d'acides aminés de la PAP humaine.

### A/ DOSAGE EXPERIMENTAL

### 1. Dosage de PAP chez des patients atteints de mucoviscidose :

**Protocole :** Des prélèvements sanguins ont été analysés chez 66 patients agés de 15 jours à 40 ans, chez qui le diagnostic de mucoviscidose avait été confirmé par le test à la sueur (Shwachman, H. et al, Ann.N.Y.Acad.Sci. 1962 93:600) et/ou l'analyse génétique (Collins, F.S. Cystic fibrosis : Molecular biology and therapeutic implications. Science 1992 256:774). Une série d'individus sains d'age correspondant a été étudiée en parallèle. La PAP a été dosée dans ces prélèvements, en utilisant le dosage suivant :

**Dosage de PAP sérique :** Ce dosage, de type ELISA compétitif, utilise des anticorps polyclonaux obtenus chez le lapin par injections répétées de PAP humaine purifiée (Keim, V. et al, Gastroenterology 1992 103:248). Des protéines de suc pancréatique humain contenant de la PAP ont été adsorbées sur plaques de microtitration (100 ng de protéines par puits). L'échantillon de sérum (50 µl) est mis dans un tube de type Eppendorf en présence de 0,5 µl de sérum immun et incubé dans un volume final de 100 µl de Tris 100 mM pH 7,4, 1 % Tween® 20, 1,5 % albumine sérique bovine, pendant 2 h à température ambiante. Le mélange est ensuite déposé dans un puits de microtitration traité comme décrit ci-dessus et incubé pendant 2 h à température ambiante. Le puits est alors rincé trois fois avec 300 µl de PBS contenant 0,5 % de Tween 20. La révélation se fait à l'aide d'un anticorps de chèvre anti-immunoglobulines de lapin, marqué à la péroxydase (100 µl à 0,2 µg/ml). La courbe de référence est établie à l'aide de PAP purifiée.

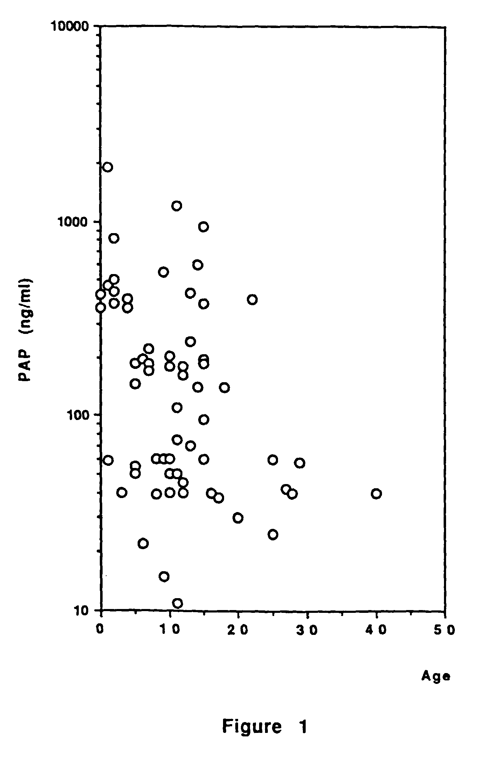

**Résultats** : Les résultats présentés à la Figure 1 montrent
* que chez les patients atteints de mucoviscidose la concentration sérique de PAP est toujours supérieure à celle des témoins. Les valeurs des témoins s'échelonnent entre 1 et 10 ng/ml, celles des patients entre 11 et 1900 ng/ml.
* que les valeurs les plus élevées sont observées chez les patients les plus jeunes.

### 2. Dosage de PAP chez les nouveau-nés : L'analyse anatomo-pathologique de foetus atteints de mucoviscidose a montré dans tous les cas une atteinte pancréatique (Boué A et al, Hum. Genet. 1988, 74:288). Les inventeurs en ont déduit que la concentration de PAP pouvait déja être élevée dans le sang des nouveaunés atteints de mucoviscidose.

**Protocole :** Ceci a pu être démontré par l'expérimentation suivante :
Le dosage de PAP a été réalisé à partir de cartons provenant de centres de dépistage, portant des échantillons de sang séché. Les cartons correspondaient à quatre groupes d'individus :
Groupe 1 : Témoins (trypsine normale à la naissance) (n = 50).
Groupes 2, 3 et 4 : Enfants présentant une trypsine élevée à la naissance.
Groupe 2 : Enfants "faux-positifs" (pas d'anomalie du gène CFTR, test à la sueur négatif). (n = 60)
Groupe 3 : Enfants hétérozygotes pour le gène CFTR muté (non atteints), test à la sueur négatif. (n = 33)
Groupe 4 : Enfants atteints de mucoviscidose (homozygotes pour le gène CFTR muté, test à la sueur positif) (n = 11).

**Dosage :** Le dosage a été réalisé de la manière suivante :
Les dépôts de sang sur les cartons utilisés dataient de moins de deux mois. Les cartons correspondaient aux normes définies par l'Association Française de Dépistage.
Un disque de 6 mm de diamètre a été découpé dans chaque carton, au niveau de la tache de sang. La quantité de sang correspondante est d'environ 10 µl.
Chaque disque a été déposé dans un tube de type Eppendorf de 1,5 ml de volume total, contenant 150 µl d'une solution de Tris 100 mM, pH 7,4, 1 % Tween® 20, 1,5 % albumine sérique bovine et soumis à agitation sur un appareil Multivortexer (Amersham France S.A.) pendant 8 h. à température ambiante. La totalité de la solution contenant le sang désorbé est ajoutée à 50 µl de solution de Tris pH 7,4, 1 % Tween® 20, 1,5 % albumine sérique bovine contenant 0,5 µl d'antisérum anti-PAP (volume total 200 µl) et incubée pendant 2 h à température ambiante. La suite du dosage est réalisée exactement comme décrit ci-dessus.

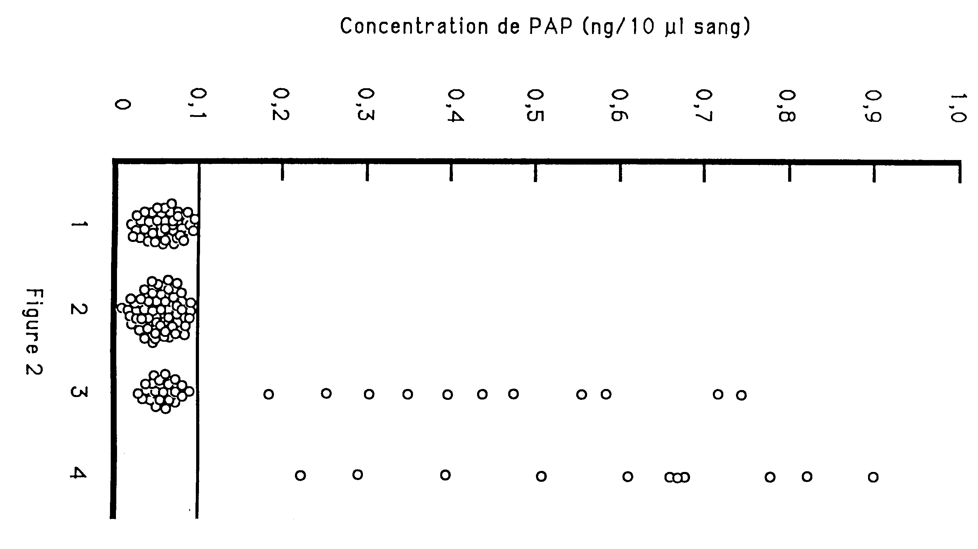

**Résultats :** Les résultats, présentés dans la Figure 2, peuvent être résumés comme suit :
1) Les enfants ayant une trypsine négative (Témoins, Groupe 1) ont une concentration de PAP sérique comprise entre 0,01 et 0,1 ng/10 µl de sang (médiane 0,065).
2) Les enfants du Groupe 2 (Trypsine positive mais non atteints) présentent les mêmes valeurs que les témoins.
3) Un tiers (11/33) des enfants du groupe 3 (Hétérozygotes) ont présenté une concentration sérique de PAP allant de 0,18 à 0,75 ng/10 µl de sang.
4) Tous les enfants atteints de mucoviscidose (11/11) ont présenté une concentration sérique de PAP comprise entre 0,22 et 0,90 ng/10 µl de sang.

### Conclusion :

1) Aucun enfant témoin n'a présenté une concentration sanguine de PAP supérieure à 0,1 ng/10 µl de sang. Cette expérience montre que toute valeur supérieure à 2 fois la médiane (supérieure à 0,11 ng/10 µl de sang) est anormale.
2) D'ailleurs, tous les enfants atteints de mucoviscidose ont montré des valeurs de PAP dans le sang supérieures à ce seuil.
3) Contrairement au système basé sur le dosage de trypsine, le dosage de PAP semble ne sélectionner que les enfants ayant une mucoviscidose et ceux, parmi les hétérozygotes pour le gène altéré, dont l'atteinte pancréatique est la plus prononcée.

Le test proposé présente donc les qualités requises pour son application au dépistage néonatal de la mucoviscidose.

### B/ DOSAGE INDUSTRIEL

### 1. A l'aide d'anticorps monoclonaux

La mise au point d'un test ELISA de type "sandwich" pour la PAP a nécessité de sélectionner deux anticorps monoclonaux, reconnaissant deux épitopes différents sur l'antigène :
. l'un destiné à revêtir les puits de plaques de microtitration (anticorps de capture),
. l'autre, couplé à un enzyme, destiné à révéler l'antigène adsorbé sur les puits (anticorps de révélation).

Cette sélection a été réalisée avec des anticorps monoclonaux produits en ascites et purifiés par chormatographie d'affinité sur colonne de protéine-A Sépharose pour les IgG ou sur colonne d'imunoadsorbant pour les IgM (anticorps monoclonal de rat anti-IgM de souris couplé à une matrice de Sépharose).

L'adsorption d'une immunoglobuline sur un support solide se traduit par des modifications conformationnelles qui peuvent entraîner une perte d'activité de la molécule. Tous les anticorps monoclonaux anti-PAP obtenus dans le laboratoire ont donc, dans un premier temps, été testés pour leur capacité, après adsorption sur des plaques de microtitration (NUNC maxisorp), à reconnaitre la PAP naturelle et recombinante. Après incubation, les complexes antigène-anticorps ont été révélés à l'aide de fragments Fab d'immunoglobulines de lapin anti-PAP conjugués à la peroxydase en utilisant comme substrat de l'enzyme de l'o-phenylènediamine.

Toutes les immunoglobulines ayant conservé cette capacité ont été retenues comme anticorps potentiels de capture et couplées à de la biotine.

Ces anticorps biotinylés ont été utilisés en tests de compétition pour sélectionner un anticorps monoclonal de révélation.

Ces tests ont été réalisés avec de la PAP (naturelle et recombinante) adsorbée sur des puits de plaques de microtitration revêtus d'IgG de lapin anti-PAP. La fixation des anticorps monoclonaux biotinylés sur l'antigène (révélée à l'aide d'un complexe avidine-POD) a été mesurée en absence ou en présence d'un exces des différents anticorps monoclonaux à tester.

Tous les anticorps monoclonaux n'entrant pas en compétition avec au moins un des anticorps biotinylés (et reconnaissant donc un épitope différent), ont été sélectionnés comme anticorps potentiels de révélation.

Ils ont ensuite été couplés à la POD sous forme de fragment Fab' avant d'être utilisés pour la mise au point de la forme finale du test ELISA de dosage de la PAP.

Cette mise au point a nécessité de rechercher le couple anticorps de capture-anticorps de révélation qui permette d'obtenir la meilleure sensibilité du test, la meilleure reproductibilité des résultats et la meilleure stabilité des réactifs dans le temps.

Deux anticorps monoclonaux ont répondu à l'ensemble de ces critères de sélection:
. l'anticorps 6F3E4 utilisé en capture,
. l'anticorps 16F4B8 utilisé en révélation.

### 2. A l'aide de sérums polyclonaux

Un dosage ELISA PAP de type sandwich adapté aux conditions de dépistage a été mis au point. Ses caractéristiques sont les suivantes :

### Production des anticorps :

L'antigène était de la PAP humaine hautement purifiée, selon la technique suivante : à partir de suc de pancréas transplanté, lyophilisé, une première séparation par chromatographie d'échange d'ions (HPLC, colonne MonoS) a permis de séparer un pic contenant la PAP et un contaminant de haut poids moléculaire. Ce pic a ensuite été résolu par tamisage moléculaire (HPLC, colonne Sephacryl® 200 HR). La PAP a alors été recueillie sous forme d'une fraction homogène. Le contrôle réalisé par électrophorèse en gel SDS et coloration à l'argent a permis de garantir une pureté supérieure à 98 %.

L'immunisation a été réalisée selon la technique habituelle du laboratoire, décrite par Keim V. et al (Gastroenterology, 1992, 103:248).

La qualité des immunsérums a été testée par utilisation en Western blots de dilutions successives.

### Construction de l'ELISA :

Il s'agit d'un ELISA sandwich polyclonal/polyclonal.

Les immunoglobulines de l'immunsérum ont été purifiées par affinité sur colonne de protéine A-Sépharose.

Les immunoglobulines destinées à la révélation ont été marquées à la biotine selon la technique habituelle connue de l'homme du métier. Le système de révélation faisait intervenir l'avidine POD.

La sensibilité du test est de 50 pg/ml.

### Résultats obtenus :

Quarante prélèvements sur carton de nouveau-nés sains ont été testés. Les concentrations de PAP y étaient toutes inférieures à 60 pg/ml. Un nouveau né atteint de mucoviscidose avait une valeur de 800 pg/ml. Six enfants (âge 3 mois-10 ans) atteints de la maladie, dont le sang a été aussi prélevé sur carton, ont montré des valeurs s'échelonnant entre 0,5 ng/ml et 1,8 ng/ml.

Ces résultats confirment les différences déjà observées avec le dosage réalisé avec les anticorps monoclonaux.

## Revendications

1. Procédé pour le dépistage in vitro de la mucoviscidose, caractérisé en ce qu'il comprend une étape de dosage de la concentration de PAP (Pancreatitis-Associated Protein) humaine dans un échantillon biologique.

2. Procédé selon la revendication 1, caractérisé en ce que le dépistage de la mucoviscidose est réalisé lors d'un test néonatal.

3. Procédé selon la revendication 1 ou 2, caractérisé en ce que l'échantillon biologique est un échantillon de sang ou de sérum.

4. Procédé selon l'une quelconque des revendications 1 à 3, caractérisé par :
- le dosage de la concentration de PAP dans l'échantillon biologique,
- la comparaison de la valeur obtenue, avec la valeur de la médiane calculée pour un groupe déterminé d'échantillons de référence préalablement soumis au dosage de la PAP, dans les mêmes conditions.

5. Procédé selon l'une quelconque des revendications 1 à 4, caractérisé par le dosage de la concentration sérique de PAP, et par :
- la mise en contact d'un échantillon biologique, par exemple le sang ou le sérum, avec des anticorps reconnaissant la PAP humaine,
- la détection de la formation d'une réaction immunologique de type PAP-anticorps,
- le dosage des complexes PAP-anticorps.

6. Procédé selon la revendication 5, caractérisé en ce que les anticorps sont des anticorps monoclonaux.

7. Procédé selon la revendication 6 caractérisé en ce que les anticorps monoclonaux sont spécifiques de la PAP humaine et sont par conséquent dépourvus de réaction immunologique avec les constituants présents dans le sang normal ou dans un échantillon biologique normal de référence et notamment en ce qu'ils sont dépourvus de réaction avec les lectines du sang.

8. Procédé selon la revendication 5, caractérisé en ce que les anticorps reconnaissant la PAP humaine sont contenus dans un sérum polyclonal et en ce que la détection de complexes PAP-anticorps est réalisée à l'aide d'un sérum polyclonal contenant des anticorps anti-PAP marqués.

9. Procédé selon la revendication 8, dans lequel les anticorps anti-PAP sont biotinylés.

10. Procédé de dépistage in vitro de la mucoviscidose selon l'une quelconque des revendications 1 à 7, caractérisé en ce qu'il comprend les étapes suivantes :
- la mise en contact d'une quantité déterminée d'un échantillon biologique, par exemple d'un échantillon de sang ou de sérum dans lequel on recherche la PAP, avec un anticorps monoclonal fixé sur un support solide (dit "anticorps de capture") reconnaissant spécifiquement la PAP humaine, dans des conditions permettant la formation d'un complexe immunologique entre l'anticorps de capture et la PAP lorsqu'elle est présente dans l'échantillon dosé,
- la mise en contact du milieu de réaction obtenu à l'étape précédente, avec un anticorps monoclonal (dit "anticorps de révélation") reconnaissant un épitope différent de l'épitope reconnu par l'anticorps de capture, l'anticorps de révélation étant marqué, dans des conditions permettant la formation d'un complexe immunologique entre l'anticorps de révélation et l'antigène préalablement lié à l'anticorps de capture,
- le lavage pour éliminer les anticorps de révélation n'ayant pas réagi,
- la détection des complexes anticorps de capture - antigène PAP - anticorps de révélation,
- la détermination de la concentration de PAP.

11. Procédé selon la revendication 10, caractérisé en ce que la concentration de PAP est comparée avec une médiane calculée pour un groupe déterminé d'échantillons de référence préalablement soumis au même dosage de la PAP dans les mêmes conditions.

12. Procédé selon la revendication 10 ou 11, caractérisé en ce que l'anticorps de révélation est coupé à la peroxydase de raifort.

13. Procédé selon l'une quelconque des revendications 10 à 12, caractérisé en ce que l'anticorps de révélation est remplacé par ses fragments F(ab')2 ou Fab'.

14. Procédé selon l'une quelconque des revendications 10 à 13, caractérisé en ce que l'anticorps de capture est l'anticorps 6F3E4 produit par l'hybridome déposé à l'ECACC sous le numéro 92122310, le 23 Décembre 1992.

15. Procédé selon l'une quelconque des revendications 10 à 13, caractérisé en ce que l'anticorps de révélation est l'anticorps 16F4B8 produit par l'hybridome déposé à l'ECACC sous le numéro 92122309, le 23 Décembre 1992.

16. Procédé selon la revendication 10, caractérisé en ce que les anticorps monoclonaux de révélation et de capture, sont remplacés par des sérums polyclonaux reconnaissant la PAP humaine.

17. Utilisation d'anticorps monoclonaux reconnaissant spécifiquement la PAP humaine ou de sérums polyclonaux reconnaissant la PAP humaine, pour le dépistage in vitro de la mucoviscidose.

## Claims

1. Procedure for the in vitro screening for mucoviscidosis, characterized in that it comprises a step in which the concentration of human PAP (pancreatitis-associated protein) is determined in a biological sample.

2. Procedure according to Claim 1, characterized in that the screening for mucoviscidosis is carried out as part of a neonatal test.

3. Procedure according to Claim 1 or 2, characterized in that the biological sample is a blood or serum sample.

4. Procedure according to any one of the Claims 1 to 3, characterized by:
- the determination of the PAP concentration in the biological sample,
- the comparison of the value obtained with the value of the calculated median for a defined group of reference samples previously subjected to the PAP determination under the same conditions.

5. Procedure according to any one of the Claims 1 to 4, characterized by the determination of the PAP concentration in serum and by :
- the placing of a biological sample, for example blood or serum, in contact with antibodies recognizing human PAP,
- the detection of the formation of an immunological reaction of the PAP- antibody type,
- the determination of the PAP-antibody complexes.

6. Procedure according to Claim 5, characterized in that the antibodies are monoclonal antibodies.

7. Procedure according to Claim 6, characterized in that the monoclonal antibodies are specific for human PAP and consequently do not show an immunological reaction with the constituents present in normal blood or in a normal biological reference sample and particularly in that they do not show a reaction with the lectins of the blood.

8. Procedure according to Claim 5, characterized in that the antibodies recognizing the human PAP are contained in a polyclonal serum and in that the detection of PAP-antibody complexes is carried out with the aid of a polyclonal serum containing labelled anti-PAP antibodies.

9. Procedure according to Cairn 8, in which the anti-PAP antibodies are biotinylated.

10. In vitro screening procedure for mucoviscidosis according to any one of the Claims 1 to 7, characterized in that it comprises the following steps:
- the placing of a defined quantity of a biological sample, for example a blood or serum sample in which the PAP is to be assayed, in contact with a monoclonal antibody fixed to a solid support (called "capture antibody") and which recognizes specifically the human PAP under conditions allowing the formation of an immunological complex between the capture antibody and the PAP when it is present in the sample being assayed,
- the placing of the reaction medium obtained in the previous step in contact with a monoclonal antibody (called "revelation antibody") which recognizes a different epitope from the epitope recognized by the capture antibody, the revelation antibody being labelled under conditions allowing the formation of an immunological complex between the revelation antibody and the antigen previously bound to the capture antibody,
- washing to eliminate unreacted revelation antibody,
- the detection of the capture antibody-PAP antigen-revelation antibody complexes,
- the determination of the PAP concentration

11. Procedure according to Claim 10, characterized in that the PAP concentration is compared with a median calculated for a defined group of reference samples previously subjected to the same PAP determination under the same conditions.

12. Procedure according to Claim 10 or 11, characterized in that the revelation antibody is coupled to horseradish peroxidase.

13. Procedure according to any one of the Claims 10 to 12, characterized in that the revelation antibody is replaced by its F(ab')2 or Fab' fragments.

14. Procedure according to any one of the Claims 10 to 13, characterized in that the capture antibody is the antibody 6F3E4 produced by the hybridoma deposited with the ECACC under the number 92122310 on 23 December 1992.

15. Procedure according to any one of the Claims 10 to 13, characterized in that the revelation antibody is the antibody 16F4B8 produced by the hybridoma deposited with the ECACC under the number 92122309 on 23 December 1992.

16. Procedure according to Claim 10, characterized in that the monoclonal capture and revelation antibodies are replaced by polyclonal sera recognizing the human PAP.

17. Use of monoclonal antibodies recognizing specifically the human PAP or polyclonal sera recognizing the human PAP for the in vitro screening for mucoviscidosis.

## Patentansprüche

1. Verfahren zur in vitro-Erkennung der Mucoviszidose, dadurch gekennzeichnet, daß es die Bestimmung der Konzentration des menschlichen PAP (pancreatitis-associated protein) in einer biologischen Probe umfaßt.

2. Verfahren nach Anspruch 1, dadurch gekennzeichnet, daß die Erkennung der Mucoviszidose als ein neonataler Test durchgeführt wird.

3. Verfahren nach Anspruch 1 oder 2, dadurch gekennzeichnet, daß die biologische Probe eine Blut- oder Serumprobe ist.

4. Verfahren nach einem der Ansprüche 1 bis 3, gekennzeichnet durch:
- die Bestimmung der Konzentration des PAP in der biologischen Probe,
- den Vergleich des erhaltenen Wertes mit dem Mittelwert, berechnet aus einer bestimmten Gruppe von Referenzproben, mit denen vorher unter den gleichen Bedingungen eine PAP-Bestimmung durchgeführt wurde.

5. Verfahren nach einem der Ansprüche 1 bis 4, gekennzeichnet durch die Bestimmung der Serumkonzentration des PAP und durch:
- das Inkontaktbringen der biologischen Probe, zum Beispiel des Blutes oder des Serums, mit Antikörpern, die das menschliche PAP erkennen,
- den Nachweis des Auftretens einer immunologischen Reaktion des Types PAP-Antikörper,
- die Bestimmung des Komplexes PAP-Antikörper.

6. Verfahren nach Anspruch 5, dadurch gekennzeichnet, daß die Antikörper monoclonale Antikörper sind.

7. Verfahren nach Anspruch 6, dadurch gekennzeichnet, daß die monoclonalen Antikörper für das menschliche PAP spezifisch sind und folglich ohne immunologische Reaktion mit den Bestandteilen, die in normalem Blut und in einer normalen biologischen Referenzprobe vorhanden sind, und insbesondere, daß sie keine Reaktion mit den Lectinen des Bluts zeigen.

8. Verfahren nach Anspruch 5, dadurch gekennzeichnet, daß die Antikörper, die menschliches PAP erkennen, in einem polyclonalen Serum enthalten sind, und daß der Nachweis der PAP-Antikörper-Komplexe mit Hilfe eines polyclonalen Serums erfolgt, das markierte anti-PAP-Antikörper enthält.

9. Verfahren nach Anspruch 8, wobei die anti-PAP-Antikörper biotinyliert sind.

10. Verfahren zur in vitro-Erkennung der Mucoviszidose nach einem der Ansprüche 1 bis 7, dadurch gekennzeichnet, daß es die folgenden Schritte umfaßt:
- das Inkontaktbringen einer bestimmten Menge einer biologischen Probe, zum Beispiel einer Blut- oder Serumprobe, in welcher das PAP gesucht wird, mit einem monoclonalen Antikörper, der auf einem festen Träger fixiert ist (d.h. einem "Einfangantikörper"), der spezifisch menschliches PAP unter Bedingungen erkennt, die die Bildung eines immunologischen Komplexes zwischen Einfangantikörpern und dem PAP erlauben, wenn es in der zu bestimmenden Probe vorhanden ist,
- das Inkontaktbringen des Reaktionsmediums, das im vorstehenden Schritt erhalten wurde, mit einem monclonalen Antikörper (d.h. einem "Entwicklungsantikörper"), der ein anderes Epitop als das Epitop, das vom Einfangantikörper erkannt wird, erkennt, wobei der Entwicklungsantikörper markiert ist, unter Bedingungen, die die Bildung eines immunologischen Komplexes zwischen dem Entwicklungsantikörper und dem Antigen, das vorher an den Einfangantikörper gebunden war, erlauben
- das Waschen, um Entwicklungsantikörper, die nicht reagiert haben, zu entfernen,
- den Nachweis der Komplexe aus Einfangantikörper-Antigen PAP-Entwicklungsantikörper,
- die Bestimmung der PAP Konzentration.

11. Verfahren nach Anspruch 10, dadurch gekennzeichnet, daß die Konzentration des PAP verglichen wird mit einem Mittelwert, der für eine bestimmte Gruppe von Referenzproben bestimmt wurde, mit denen vorher die gleiche Bestimmung des PAP unter gleichen Bedingungen durchgeführt wurde.

12. Verfahren nach Anspruch 10 oder 11, dadurch gekennzeichnet, daß der Entwicklungsantikörper an Meerrettichperoxidase gebunden ist.

13. Verfahren nach einem der Ansprüche 10 bis 12, dadurch gekennzeichnet, daß der Entwicklungsantikörper durch seine Fragmente F(ab')₂ oder Fab' ersetzt ist.

14. Verfahren nach einem der Ansprüche 10 bis 13, dadurch gekennzeichnet, daß der Einfangantikörper der Antikörper 6F3E4 ist, der von dem Hybridom produziert wird, das unter der Nummer 92122310 am 23. Dezember 1992 bei der ECACC hinterlegt wurde.

15. Verfahren nach einem der Ansprüche 10 bis 13, dadurch gekennzeichnet, daß der Entwicklungsantikörper der Antikörper 16F4B8 ist, der von dem Hybridom produziert wird, das unter der Nummer 92122309 am 23. Dezember 1992 bei der ECACC hinterlegt wurde.

16. Verfahren nach Anspruch 10, dadurch gekennzeichnet, daß die monclonalen Entwicklungs- und Einfangantikörper ersetzt sind durch polyclonale Seren, die das menschliche PAP erkennen.

17. Verwendung der monoclonalen Antikörper, die spezifisch menschliches PAP erkennen, oder der polyclonalen Seren, die menschliches PAP erkennen, zur in vitro-Erkennung der Mucoviszidose.
